# EUROPEAN PATENT APPLICATION

(11) **EP 3 203 400 A1**
(43) Date of publication of application: **09.08.2017**
(21) Application number: 16382045.9
(22) Date of filing: 03.02.2016
(51) Int. Cl.: G06F 19/00

(54) **A COMPUTER IMPLEMENTED METHOD OF GENERATION OF STATISTICALLY UNCORRELATED MOLECULE'S CONFORMATIONS AND COMPUTER PROGRAMS**

(71) Applicant: Universitat Rovira I Virgili, 43003 Tarragona (ES)
(72) Inventor: GUO, Yachong, 43007 Tarragona (ES); BAULIN, Vladimir, 43893 Altafulla (Tarragona) (ES)
(74) Representative: Juncosa Miro, Jaime

(57) **Abstract**

The method is executed in a computer system and comprises performing a SAW conformations generation of Monte Carlo simulations of at least one molecule comprising a plurality of subunits by using a GPU with a CUDA library. A SAW molecular conformations generation is performed by implementing a Rosenbluth algorithm including allocating individual data of each molecular configuration W in a GPU memory for the Monte Carlo simulation, and generating said molecular configuration W of each individual data by using a stream processing in the GPU, wherein said stream processing starts with the initialization of the GPU and the initialization of a random generator for the GPU, so that that each thread of the stream processing generates statistically uncorrelated random numbers, and each thread of the stream processing is performed simultaneously and synchronously generating its own statistically uncorrelated conformation of the molecule using the Rosenbluth algorithm.

## Description

### Technical Field

The present invention is directed, in general, to the field of computer systems and methods. In particular, the invention relates to a computer implemented method, and computer program products, of generation of statistically uncorrelated molecule's conformations. Principally, the invention is based on a CUDA-platform-based parallel implementation with a graphical processor unit (GPU) via a Rosenbluth algorithm. The method includes allocating individual data of each molecular configuration in a GPU memory for a Monte Carlo simulation thereof, and generating the molecular configuration using stream processing in the GPU.

According to the present invention following definitions are to be taken into account:
- Monte Carlo simulation: refers to a stochastic technique based on theory of probabilities that uses repeated random molecular conformations to obtain numerical results. This method relies on generation of large number of statistically uncorrelated molecular conformations. Usually Monte Carlo technique is applied (but not limited) to molecular simulations to obtain the properties of molecular systems. In particular, Monte Carlo methods are used to study interacting molecules between each other, and complex and cooperative behavior of the system. Although certain approximation is usually introduced into the models, the computational time remains to be a major challenge for the sake of the balance between the accuracy of system and efficiency.
- Molecule: is composed of subunits (group of atoms) of different sizes and types with a given connectivity and angular restrictions. For instance, a molecule may be a polymer or a small solute molecule, among others.
- Subunit (group of atoms, for instance a monomer when the molecule is a polymer): is the minimal indivisible part of a molecule.
- Conformation of a molecule: set of coordinates of each subunit. Translational or rotational invariance of certain conformations can be considered as different conformations or the same depending on a problem.
- Self-avoiding walk (SAW): is a sequence of moves in space that does not visit the same point more than once. The walking path is composed of subunits of different sizes and types with a given connectivity and angular restrictions.
- Rosenbluth method/algorithm: is a Monte Carlo method of generation correctly distributed molecular configurations and calculation of statistical weights. Rosenbluth algorithm have been widely applied and used due to its efficiency, simplicity to program. The algorithm can generate either equally probable configurations or configurationally-biased sampling. The basic idea of Rosenbluth sampling is to avoid self-intersections by only sampling from the steps that lead to self-avoiding configurations. In this way, the algorithm only terminates if the walk is trapped in a dead end and cannot continue growing. While this still happens exponentially often, Rosenbluth sampling can produce substantially longer configurations than simple sampling. Configurations generated with Rosenbluth sampling are generated with different probabilities.
- Graphics Processing Units (GPUs): is a computer chip designed to handle image-rendering purpose, which can also perform rapid mathematical calculations.
- The Compute Unified Device Architecture (CUDA): is a parallel computing platform created by GPU Company NVIDIA. The Instruction set architecture and the parallel calculation engine make it possible to solve many complicated and time consuming computer problems and greatly shorten the calculation time.

### Background of the Invention

Computer simulation is a fundamental tool in understanding many-body system in physics and chemistry. In particular, Monte Carlo methods to study in detail how individually the component interacting with each other, the complex and cooperative behavior of the system. Although certain approximation is usually introduced into the model, the computational time remains to be a challenge for the sake of the balance between the accuracy of system and efficiency.

Microprocessors based on Central Processing Unit (CPU) have gained a rapid performance increases and cost reduction in the past century. However, this boost has slowed down due to the energy consumption and heat dissipation issues resulting into the limitation of clock frequency and the efficiency of productive activities. Industry talked this problem with two main trajectories: multi-core and the many-thread.

The industry of Graphics Processing Units (GPUs) has been kept evolving driven by fast-growing video game market in the past decade. At the same time, more and more scientists have also motivated to use GPU as an alternative to parallel, computationally expensive calculation apart from CPU. The trend is driven by the large performance gap between many threads GPU and multi-core CPU. A current example is the NVIDIA Tesla K40 can reach about 4 trillion floating-point operations per second, while a new released Intel Core i7-5960K (Haswell) processor can only reach 350 billion floating-point operations per second. Many scientific applications including quantum chemistry, molecular dynamics, computational fluid dynamics, finite element analysis, and 25 computational modeling have been accelerated by factors between 9 and 130 by running on GPUs.

US-B2-8073658 discloses a method of performing a Monte Carlo analysis using a GPU of a computer system. Individual data sets to be analyzed are allocated to respective pixel locations in a GPU memory for Monte Carlo simulation and the outcome of the Monte Carlo simulation is calculated for each data set using stream processing in the graphical processor unit. Contrary to the proposed method, in the method disclosed in this US patent a Rosenbluth sampling method is not used for generating distributed Self-avoiding walk (SAW).

CN-A-104317655 relates to a method for simulating multi-level Monte Carlo path, based on a GPU cluster, in the biological medical engineering field. The method involves allocating computing task to each node of host client nodes, based on MPI information transfer protocol. The secondary distribution task is calculated within a GPU client device, based on the computing tasks assigned to each node. The data exchange between the primary and secondary nodes are performed, based on the computing task assigned to the GPU client device. The transmission process of photons corresponding track is assigned to each of the client node.

US-B2-8756264 relates to a method of generating pseudo-random numbers on a parallel processing system that comprises generating a plurality of sub-streams of pseudo-random numbers, wherein the sub-streams are generated in parallel by one or more co-processors, and providing the plurality of sub-streams to respective processing elements, wherein the respective processing elements employ the plurality of sub-streams to execute an application. The method proposed in this invention does not relate either to a Rosenbluth sampling method for generating a distributed SAW.

New methods are therefore needed to improve parallel performance and simulation efficiency in Monte Carlo simulations performed on GPU structure, in particular by means of the Rosenbluth algorithm in order to greatly shorten the simulation time in static Monte Carlo simulation.

### Description of the Invention

Embodiments of the present invention provide according to an aspect a computer implemented method of generation of statistically uncorrelated molecule's conformations. The proposed method is executed in a computer system including one or more processors and comprises performing, by said computer system, a SAW conformations generation of Monte Carlo simulations of at least one molecule having a plurality of subunits by using a GPU with a CUDA library.

The proposed method characteristically performs said SAW molecular conformations generation by implementing a Rosenbluth algorithm that allocates individual data of each molecular configuration W in a GPU memory for the Monte Carlo simulation, and that generates the molecular configuration W of each individual data by using a stream processing in the GPU. According to the proposed method, the stream processing starts with the initialization of the GPU and the initialization of a random generator for the GPU, so that that each thread of the stream processing generates statistically uncorrelated random numbers. In addition, each thread of the stream processing is performed simultaneously and synchronously generating its own statistically uncorrelated conformation of the molecule using the Rosenbluth algorithm.

According to the proposed method, and before said initialization of the GPU is performed, a configuration step that includes the selection of a coordinate system and a space dimension for each of the subunits of said molecule to be simulated is executed, in a 2D or a 3D dimension. Moreover, said configuration step further discretizes the selected space dimension into a real space or a lattice space.

In addition, the architecture of the molecule and molecular parameters thereof, including the number of composing subunits, the size and type of subunits, the connectivity of subunits, bond lengths and optionally restrictions on the angles, are further defined by a user.

Preferably, the initialization of the GPU involves a complete initialization executed without any memory pre-consumption.

According to an embodiment, the selected coordinate system and space dimension and the defined architecture and molecular parameters are stored in a constant memory of GPU. Alternatively, the selected coordinate system and space dimension and the defined architecture and molecular parameters may be stored in a GPU global memory.

Moreover, the execution of each thread of the coordinates of each subunit and of the corresponding Rosenbluth weight may be performed in a shared memory of GPU, and optionally also in the global memory of GPU.

At the end of the execution of each thread, the coordinates of each subunit and a corresponding Rosenbluth weight are stored in a CPU memory.

According to a preferred embodiment, the stream processing in the GPU comprises:
a) defining (e.g. by said user) a total number of blocks as a result of dividing the total number of conformations to be generated by a given block dimension and allocating to the GPU shared memory each of the blocks;
b) placing a first subunit in a center of the coordinate system;
c) placing a next subunit nearby the first subunit according to a random chosen direction in the coordinate system, wherein said random chosen direction being:
   c1) a random angle in the real space implementation; or
   c2) a random neighboring cell in the lattice implementation;
d) performing a checking of the position of said next subunit in order to avoid overlap with the first subunit, and depending on the result:
   d1) in case of overlap the move to said position is rejected and said step c) is repeated; or
   d2) in case of no overlapping said step b) is repeated providing a chain generation; and
e) checking whether a free space is available in the molecule configuration and in case no free space being available terminating the stream processing, and then restart from step b) until all the subunits have been placed.

According to an embodiment, the implementation of step c) for a next and subsequent subunits involves performing a bias to position each new subunits taking into account a calculation of the Rosenbluth weight representing the probability of positioning a new subunit without overlapping with previous subunits, being said Rosenbluth weight calculation done by means of:
- for the real space implementation during the chain generation, steps c) to d) are repeated Ntrial times and the fraction of successful positions involving no overlap is counted in Nallowed, wherein if Nallowed>0, a new position is accepted with the weight 1/Nallowed, and the weight of the configuration W is multiplied by the factor 1/Nallowed; or
- for the lattice implementation the Rosenbluth weight is calculated after the whole chain is generated.

According to another embodiment in lattice and real space implementation, step c) is performed according to a configurational-bias sampling generation and includes the calculation of a probability of placing subunits according to a given distribution, the Rosenbluth weight of the molecular configuration W being corrected by said calculated probability.

Other embodiments of the invention that are disclosed herein include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

Therefore, present invention discloses a CUDA-platform-based parallel implementation of the Rosenbluth molecular generation algorithm outperforming by a factor of up to 1900 times an equivalent implementation on single CPU processor. This boost will greatly shorten the simulation time in static Monte Carlo simulation related with Rosenbluth algorithm. With growing popularity and performance of GPU structure, the proposed method can be applied on different platforms and operating system serve a huge benefits in simulation efficiency with the same accuracy compared with CPU calculation. The present invention breaks the performance bottleneck of existing molecular conformation generating methods, significantly improving the parallel performance, and has broad application prospects in the static Monte Carlo simulation of high resolution.

### Brief Description of the Drawings

The previous and other advantages and features will be more fully understood from the following detailed description of embodiments, with reference to the attached figures, which must be considered in an illustrative and non-limiting manner, in which:
Fig. 1 is a flow diagram illustrating the structure of the parallel generation of SAW conformations using the Rosenbluth algorithm.
Fig. 2 is flow diagram illustrating said Rosenbluth algorithm of generation of SAW conformations using GPU.
Fig. 3 is a schematic example of the memory structure and data flow between global memory, shared memory and threads of GPU.

### Detailed Description of the Invention and of Preferred Embodiments

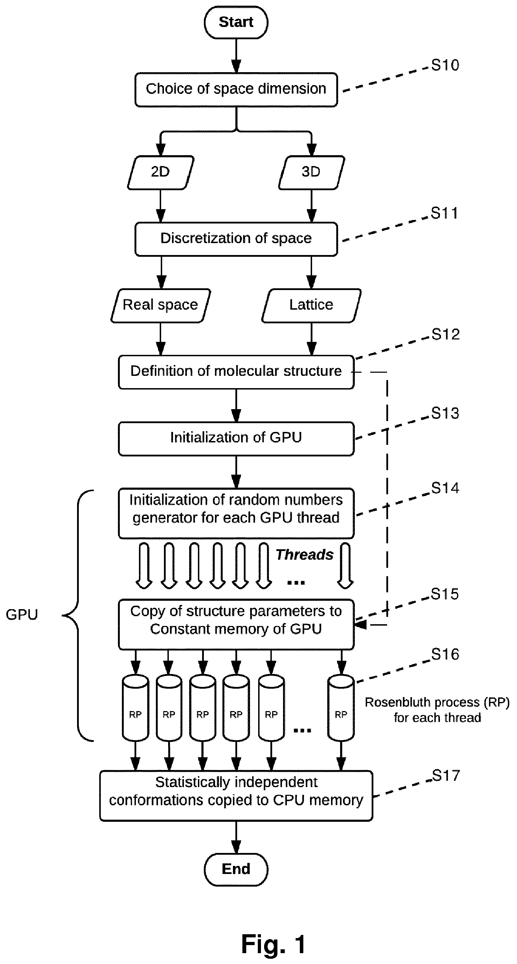

Fig 1 illustrates the process of generation of SAW conformations using Rosenbluth algorithm implemented in GPU. According to the proposed method, in Step S10, a configuration step is previously executed in which the coordinate system and the space dimension are chosen. Since the coordinates of each subunit define the conformation, the coordinate system and the space dimension have to be chosen: 2D, 3D, etc. In addition, Step S11, the discretization of space is defined: real space or lattice (cubic, triangular, square, honeycomb, etc.). In real space there is no restriction on bond lengths, angles, coordinates because the variables are continuous. On a lattice the configurational space is restricted by the geometry of a particular lattice and thus, the coordinates, the angles, the bond lengths are discretized.

Then, in Step S12, architecture of a molecule and molecular parameters thereof are defined, e.g. by a user (not illustrated), within the choice of the space dimension, the discretization of space and geometry: the number of composing subunits, the size and type of subunits, the connectivity of subunits, bond lengths and optionally, restrictions on the angles.

In Step S13 the work (i.e. the stream processing) in GPU starts with the initialization of GPU and the initialization of random generator for GPU. It is done in such a way that each thread generates statistically uncorrelated random numbers. Preferably, for good performance, GPU is completely initialized without any memory pre-consumption.

Then, in step S14 a proper random number generator is chosen. Random number generators are implemented in different realizations included in CUDA library by NVIDIA, for example XORWOW algorithm, Mersenne Twister series. Each thread generates its own random generator simultaneously and can be used on the fly.

At that time, in Step S15, the selected coordinate system and space dimension and the defined molecular architecture parameters may be copied into a GPU constant memory. Instead, it can be copied into GPU global memory; however this will be less effective reducing the performance.

Excluded the initialization of GPU, steps S12 and S14 can switch.

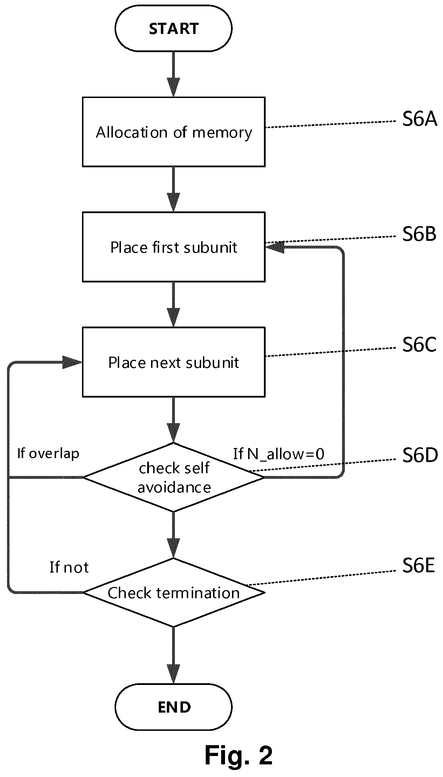

In step S16 each thread of the stream processing simultaneously and synchronously generates its own statistically uncorrelated conformation of the molecule using the Rosenbluth algorithm as shown on Fig 2. The coordinates of the conformation and the corresponding Rosenbluth weight are first stored in the shared memory of GPU during the generation in each GPU block, and then copied to global memory of GPU when the configuration is generated. In the end of this process they can be copied back to CPU memory in step S17 or they can also be processed on the fly during the generation process depending on the purpose of the user.

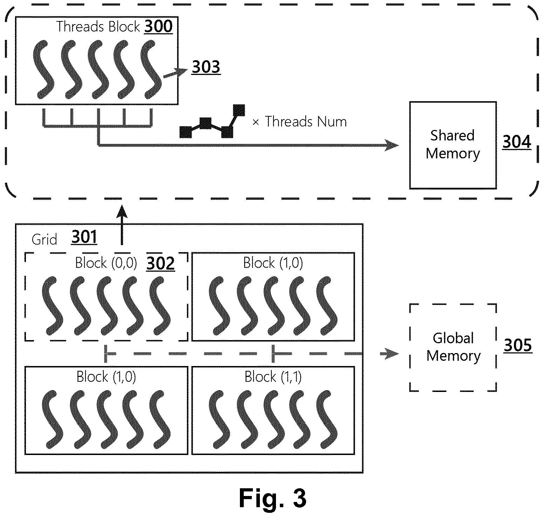

With reference now to Fig. 2, therein it is illustrated the Rosenbluth algorithm executed on each thread on GPU. In the beginning, the structure of the GPU memory (see Fig. 3) is defined as in step S6A. D denotes the dimension of the space; blockdim.x is the block dimension defined by user and chainlength is the total number of conformations. With this, the number of D arrays with dimensions blockdim.x*chainlength are allocated in the GPU shared memory to store the coordinates. Same numbers of arrays with dimensions blockdim.x are allocated in the GPU shared memory to store a temporary position of the subunit during molecule generation. The variables, such as Rosenbluth weight, increment counter for overlap which depends on the thread index, are stored in the GPU shared memory; each with size of blockdim.x to distinguish between different configurations.

The total number of blocks is defined as the total number of configurations to be generated divided by the block dimension. Thus, each generated configuration is tagged by the thread number in each block. All the coordinates and weights associated with each chain are stored in the GPU shared memory.

In Step S6B, the first subunit is placed in the center of the coordinate system.

Then, in step S6C, a random direction (random angle in real space realization or random neighboring cell in a lattice realization) of a second and subsequent subunit is chosen. In case of a biased Rosenbluth generation method, instead of random direction, the direction in the field is chosen. The bond length dictates a position of a second subunit in the chosen direction.

In step S6D the subunit position is checked for overlaps with all previous subunits. If there is an overlap with at least one of previous subunits, the move to this position is rejected and the process of selection of the new direction step S6C is repeated.

Starting from the 3rd subunit, a bias is introduced to position the new subunit because of the new subunit may overlap with the previous subunits. This is taken into consideration in the so-called Rosenbluth weight, which is by definition the probability of positioning a new subunit without overlapping with previous subunits.

The process to calculate the Rosenbluth weight is different for a real space and on lattice realizations. On lattice, the probability can be easily calculated by looking into the occupancy of previous generated subunits since the number of neighbors of the lattice cell is given by the geometry of the lattice (so-called lattice number). Thus, on a lattice the Rosenbluth weight is calculated after the whole chain is generated to avoid the unnecessary computation. In a real space, this process is different since the space is continuous. During the chain generation, steps S6C-S6D are repeated Ntrial times and the fraction of successful positions (no overlap) is counted in Nallowed. If Nallowed>0, a new position is accepted with the weight 1/Nallowed, the weight of the configuration W is multiplied by the factor 1/Nallowed.

If Nallowed=0 the process of random generation is interrupted by a situation when a next subunit cannot be placed (for example, all possible spaces are occupied by previous subunits), the current configuration is discarded. This will terminate the generation of all conformations in the whole block and restart it again in step S6B.

Fig 3 is a block diagram illustrating a GPU in accordance with some embodiments. Computation is performed by sets of streaming multiprocessors, each containing several computer units. Code is executed as a block 300/302 of threads 303 on a particular multiprocessor. Blocks 300/302 of threads 303 are grouped into a grid 301. Each multiprocessor contains a small shared memory store that can be accessed by all threads in a given block 300/302. For the current embodiments the user will define the size of the block 300/302. The total number of blocks 300/302 or the grid will automatically be defined as the total number of chains dividing the block dimension. On a single block 300/302, each thread 301 simultaneously and synchronously generates its own statistically uncorrelated conformation of the molecule using the Rosenbluth algorithm. All the data will be stored into shared memory during the generation process. The data on the shared memory can also be used on the fly when running the method and deleted. It can also be saved to global memory 305 on GPU and later copied back to CPU.

In order to compare the validity and the performance of the proposed GPU implementation, a CPU version of the same algorithm was implemented to test the performance of GPU version.

A direct comparison between CPU and GPU is presented in Table 1. A speed-up factor is defined as follows. tCPU is the execution time on a single CPU core and tGPU is the runtime on GPU. The run contains one million conformations sampling generated with different number of subunits completed either on NVidia Tesla K80 GPU or Intel i5-3320m CPU, Nallowed is set to 200. For the lattice version, the algorithm finds that GPU outperforms the single CPU 1900 times for the smallest molecule lengths consisting of 4 subunits. With longer molecules, the efficiency gradually decreases. However, GPU is still faster than CPU by a factor of 172 for a molecule consisting of 64 subunits. The decrease comes mainly from the increasing consumption of the shared memory of each block and is limited by shared memory size and thus hardware limitations, not the method itself.

**Table 1 Comparison of the calculation speed between CPU and GPU version of code**

| Molecule Length | Time for 1 mln chain / s | | Speed up /times |
|---|---|---|---|
| | tGPU | tCPU | |
| 4 | 0.00676 | 12.848 | 1900.592 |
| 8 | 0.01789 | 31.267 | 1747.736 |
| 16 | 0.06670 | 88.854 | 1332.144 |
| 24 | 0.17810 | 132.406 | 743.436 |
| 32 | 0.41466 | 192.643 | 464.580 |
| 48 | 1.19394 | 342.454 | 286.826 |
| 64 | 3.17465 | 546.526 | 172.153 |

The proposed invention may be implemented in hardware, software, firmware, or any combination thereof. If implemented in software, the functions may be stored on or encoded as one or more instructions or code on a computer-readable medium.

Computer-readable media includes computer storage media. Storage media may be any available media that can be accessed by a computer. By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media. Any processor and the storage medium may reside in an ASIC. The ASIC may reside in a user terminal. In the alternative, the processor and the storage medium may reside as discrete components in a user terminal.

As used herein, computer program products comprising computer-readable media including all forms of computer-readable medium except, to the extent that such media is deemed to be non-statutory, transitory propagating signals.

The scope of the present invention is defined in the following set of claims.

## Claims

1. A computer implemented method of generation of statistically uncorrelated molecule's conformations, said method being executed in a computer system and comprising performing a SAW conformations generation of Monte Carlo simulations of at least one molecule comprising a plurality of subunits by using a GPU with a CUDA library, **characterized in that** it comprises performing said SAW molecular conformations generation by implementing a Rosenbluth algorithm including allocating individual data of each molecular configuration W in a GPU memory for the Monte Carlo simulation, and generating said molecular configuration W of each individual data by using a stream processing in the GPU, wherein:
- said stream processing starts with the initialization of the GPU and the initialization of a random generator for the GPU, so that that each thread of the stream processing generates statistically uncorrelated random numbers, and
- each thread of the stream processing is performed simultaneously and synchronously generating its own statistically uncorrelated conformation of the molecule using the Rosenbluth algorithm.

2. The computer implemented method of claim 1, comprising, before said initialization of the GPU, executing a configuration step that includes the selection of a coordinate system and a space dimension for each of the subunits of said molecule to be simulated, in a 2D or a 3D dimension.

3. The computer implemented method of claim 2, wherein said configuration step further comprises discretizing the selected space dimension into a real space or a lattice space.

4. The computer implemented method of claim 3, further comprising defining an architecture of the molecule and molecular parameters thereof including the number of composing subunits, the size and type of subunits, the connectivity of subunits, bond lengths and optionally restrictions on the angles.

5. The computer implemented method of claim 1, wherein said initialization of the GPU involves a complete initialization executed without any memory pre-consumption.

6. The computer implemented method of claim 4, wherein said selected coordinate system and space dimension and said defined architecture and molecular parameters are stored in a GPU constant memory.

7. The computer implemented method of claim 4, wherein said selected coordinate system and space dimension and said defined architecture and molecular parameters are stored in a GPU global memory.

8. The computer implemented method of claim 6 or 7, comprising performing the execution of each thread of the coordinates of each subunit and of the corresponding Rosenbluth weight in a GPU shared memory, and optionally a further storage in the GPU global memory.

9. The computer implemented method of claim 8 or 9, further comprising, at the end of the execution of each thread, storing the coordinates of each subunit and a corresponding Rosenbluth weight in a CPU memory.

10. The computer implemented method of claim 3, wherein said stream processing in the GPU comprises:
a) defining a total number of blocks as a result of dividing the total number of conformations to be generated by a given block dimension and allocating to a GPU shared memory each of the blocks;
b) placing a first subunit in a center of the coordinate system;
c) placing a next subunit nearby the first subunit according to a random chosen direction in the coordinate system, wherein said random chosen direction being:
c1) a random angle in the real space implementation; or
c2) a random neighboring cell in the lattice implementation;
d) performing a checking of the position of said next subunit in order to avoid overlap with the first subunit, and depending on the result:
d1) in case of overlap the move to said position is rejected and said step c) is repeated; or
d2) in case of no overlapping said step b) is repeated providing a chain generation; and
e) checking whether a free space is available in the molecule configuration and in case no free space being available terminating the stream processing, and then restart from step b) until all the subunits have been placed.

11. The computer implemented method of claim 10, wherein the size of each block is defined by a user.

12. The computer implemented method of claim 10, wherein said performed SAW molecular conformation generation is stored in the GPU shared memory or in a CPU memory.

13. The computer implemented method of claim 10, wherein the implementation of step c) for a next and subsequent subunits involves performing a bias to position each new subunits taking into account a calculation of the Rosenbluth weight representing the probability of positioning a new subunit without overlapping with previous subunits and wherein said Rosenbluth weight calculation is done:
- for the real space implementation during the chain generation, steps c) to d) are repeated Ntrial times and the fraction of successful positions involving no overlap is counted in Nallowed, wherein if Nallowed>0, a new position is accepted with the weight 1/Nallowed, and the weight of the configuration W is multiplied by the factor 1/Nallowed; or
- for the lattice implementation the Rosenbluth weight is calculated after the whole chain is generated.

14. The computer implemented method of claim 10, wherein the implementation of step c) for a configurational-bias sampling generation includes the calculation of a probability of placing subunits according to a given distribution, the Rosenbluth weight of the molecular configuration W being corrected by said calculated probability.

15. A computer program product comprising code instructions that when executed in one or more processors of a computer system implement the method steps of any of the claims 1 to 14.
